Europäisches Patentamt

European Patent Office

Office européen des brevets

(19)

(11) Veröffentlichungsnummer : **0 366 678 B1**

(12) # EUROPÄISCHE PATENTSCHRIFT

(45) Veröffentlichungstag der Patentschrift :
23.10.91 Patentblatt 91/43

(51) Int. Cl.⁵ : **A61B 17/60**

(21) Anmeldenummer : **88905179.3**

(22) Anmeldetag : **02.06.88**

(86) Internationale Anmeldenummer :
**PCT/DE88/00323**

(87) Internationale Veröffentlichungsnummer :
**WO 88/10099 29.12.88 Gazette 88/28**

(54) RINGFIXATEUR ZUR EINRICHTEN, BEFESTIGEN UND REGULIEREN DER SPANNLAGE VON KNOCHENABSCHNITTEN.

(30) Priorität : **19.06.87 DE 3720242**

(43) Veröffentlichungstag der Anmeldung :
**09.05.90 Patentblatt 90/19**

(45) Bekanntmachung des Hinweises auf die
Patenterteilung :
**23.10.91 Patentblatt 91/43**

(84) Benannte Vertragsstaaten :
**AT BE CH DE FR GB IT LI LU NL SE**

(56) Entgegenhaltungen :
**EP-A- 0 146 872**
**EP-A- 0 190 990**
**EP-A- 0 194 187**
**WO-A-85/03449**

(56) Entgegenhaltungen :
**DE-A- 2 832 631**
**DE-A- 3 345 276**
**DE-A- 3 510 305**
**FR-A- 2 405 063**
**FR-A- 2 536 984**
**US-A- 4 584 995**
**US-A- 4 615 338**

(73) Patentinhaber : **SCHEWIOR, Thomas**
**Fasanenweg 10**
**W-6903 Neckargemund (DE)**

(72) Erfinder : **SCHEWIOR, Thomas**
**Fasanenweg 10**
**W-6903 Neckargemund (DE)**

(74) Vertreter : **Mierswa, Klaus, Dipl.-Ing. et al**
**Friedrichstrasse 171**
**W-6800 Mannheim 24 (DE)**

EP 0 366 678 B1

## Beschreibung

### Technisches Gebiet:

Die Erfindung betrifft einen Ringfixateur zusammengesetzt aus Teilen eines Bausatzes zum Einrichten, Befestigen und Regulieren der Spannlage von Knochenabschnitten gemäß dem Oberbegriff des Anspruchs 1.

### Stand der Technik:

Derartige Vorrichtungen sind als sogenannte Kompressions- und Distraktionsapparate vor allem durch G.A. Ilisarov und das UdSSR-Patent No. 5387103338801/28-13 sowie das italienische Patent No. 47890-A/82 bekanntgeworden. Es handelt sich dabei um Fixateure aus Ringen und/oder Ringabschnitten, bei denen in jeder Ring- oder Ringabschnittsebene sich paarig kreuzende oder auch singuläre Spanndrähte zur Knochenfixation angeordnet sind, wobei die verschiedenen Ringe durch Stangen miteinander verbunden und die Ringebenen-Abstände durch unterschiedlich lange oder teleskopisch in der Länge verstellbare Stangen eingestellt werden. Diese Knochenfixationen mittels eines zyindrischen Rahmens und transossärer Spanndrähte nach Ilisarov haben sich gegenüber polygonalen Rahmenfixateuren oder paraaxial zum Knochen gelagerten Stabfixateuren als überlegen erwiesen. Transossäre Ring-Draht-Fixationen können, um die wesentlichen Vorteile zu nennen, in vielen Situationen ambulant und in örtlicher Betäubung angelegt werden. Es können mit ihnen in stereognostisch übersichtlicher Weise dreidimensionale Knocheneinrichtungen vorgenommen und ebenso übersichtlich sekundäre Richtarbeiten ausgeführt werden. Ohne Gefährdung der Ernährungslage der Gewebe und ohne das Risiko der Stabilitätsschwächung können beliebige Drähte bei Bedarf auch nachträglich angebracht, entfernt oder ausgetauscht werden. Die Behandlung in derartigen Ring-Draht-Fixateuren ist im Gegensatz zu den starr verankernden Stab- und Rahmenfixationen als prolongierter, kontinuierlich fortschreitender Operationsprozeß angelegt. Mißerfolge, die auf eine zu einem bestimmten Zeitpunkt erfolglos oder fehlerhaft ausgeführte Operation bezogen werden müßten, sind durch das Verfahrensmerkmal einer prozessual stets verfügbaren Korrekturmöglichkeit verfahrensfremd. Die Ilisarov'schen Ringfixationstechniken ordnen sich weitgehend dem biologischen Heilungsprozeß unter, da methoden-gemäße frühzeitige Belastungen der Gliedmaßen die von der selbstorganisatorisch-autoregulativ arbeitenden Natur auswertbaren Prozeßinformationen der Kallusbildung und der Knochentransformation darstellen. Wird doch der Kraftfluß bei derart konzentrisch gelagerten Frakturen oder Osteotomien beanspruchungsspezifisch erhalten, woraufhin die Natur im Zuge selbstorganisatorisch-autoregulativer Konsolidierung örtlich-selektiv nach Druck- und Zugbelastbarkeit differenzierbare Baumaterialien in der Fraktur- bzw. Osteotomiezone zur Ausbildung bringt und beanspruchungsspezifisch anordnet. Dadurch, daß die Ringfixateure als äußere Stabilisierungs-gerüste durch ihren zylindrischen Aufbau stauch-, biege- und torsionsstabil sind und biegbaren knochenpenetrierenden Drähten als sehr stabile Verankerungslager dienen, können bestimmte, von dosierbarer Biegsamkeit der Drähte ausgehende Gewebsbildungs- und Umbildungsvorgänge im Rahmen physiologischer Reaktionsbreite angeregt werden, und es können somit reparative und generativ neue Knochenformationen therapeutisch zielsicher gesteuert werden.

Die Ilisarov'schen Ringapparate sind prinzipiell für die meisten stereometrischen Situationen aufbaubar. Jedoch sind viele Form- und Spannungsveränderungen nicht mit genügender Präzision und im Hinblick auf die Häufigkeit der nötigen Korrekturschritte nicht mit der wünschenswerten Einfachheit und Zeitersparnis ausführbar. Auch ist bei den zwangsläufig inkonstanten Hantierungen an den originalen Ringfixateuren und den verschiedenen Nachbildungen die für den prozessualen Heilungsvorgang nötige Stetigkeit dieser extern und dynamisch fixierenden Osteosynthesetherapie nicht zuverlässig zu gewährleisten.

Ein in seiner Manövrierfähigkeit verbesserter Ringfixateur ist durch die DE-OS 34 39 795 bekanntgeworden, die eine Vorrichtung zum Einrichten von Knochenabschnitten und/oder -fragmenten bezeichnet, bei der die Verbindungsstangen zwischen den Ringen innerhalb vorbestimmter, aber geringer Grenzen allseitig schwenkbar an den Ringen gelagert und in jeder Schwenkstellung arretierbar sind, wobei die Abstände zwischen den benachbarten Ringen in verschiedenen Ebenen unabhängig von den eingestellten Schwenklagen der Stangen einstellbar sind, wobei jedoch nicht der gesamte Umfang der Ringe zur Lokalisation der Verbindungsstangen zur Verfügung steht, sondern nur auf bestimmte Ringumfangsorte beschränkt ist, die durch eine Reihe von Bohrungen vorgegeben sind. Mit diesem Apparat ist eine teilweise dislokationsfreie Kräfteneutralisierung möglich, jedoch genügt derselbe trotz seiner verbesserten dreidimensionalen Anpassungsmöglichkeit nicht ausreichend den Bedingungen einer an der natürlichen Kausalhistogenese orientierten Operationstechnik. Erstens sind die Ringe wie bei den russischen Geräten transversal liegende Flächenausschnitte von störend großem äußerem Umfang. Zweitens können die Verbindungsstangen zwischen zwei Ringen nur an fest vorgegebenen Orten der Ringe lokalisiert werden, wodurch eine völlig freie Verschieblichkeit der Enden der Verbindungsstangen auf den Ringen nicht möglich ist. Drittens ist die räumliche Drahtlenkung für die Spanndrähte des bekannten Apparates ungenügend, weil die Spanndrähte praktisch nur in Durchmesserrichtung gespannt werden können, und außermittige Drahtlagen mit einer riskanten Abknickung der Drähte verbunden sind. Viertens braucht man zum Spannen und Nachspannen der Drähte besondere Drahtspanner, die für den Spannungsvogang extra angelegt werden müssen. Fünftens gelingt es mit dem bekannten Ringfixateur nicht, die Verbinddungsstangen als drehmomentfrei axial lagernde Verankerungsorte für zwischen den Ringebenen einzuspannende Drähte zu verwenden. Sechstens besteht nur die Möglichkeit zu reibschlüssiger und nicht linienschlüssiger Klemmbefestigung in den Schwenklagern der Stangen.

Durch die EP-A3-0146872 ist eine Vorrichtung zum Einrichten von Knochenabschnitten und/oder Knochenfragmenten bekanntgeworden, die aus durch Stangen und Befestigungsmittel in ein- und verstellbaren Abständen miteinander verbindbaren Stellringen und aus Spanndrähten besteht, die sich in den von den Stellringen aufgespannten Ebenen erstrecken und mit ihren Enden mittels Halterungen an voneinander entfernten Teilen jeweils eines Stellrings unter Längsspannung befestigbar sind. Die Befestigung der Spanndrahtenden an den Stellringen dienen nachstellbar Spannelementen. Die Stangen sind in den Befestigungselementen zum Verbinden benachbarter Stellringe stufenlos axial beweglich und unabhängig davon innerhalb vorbestimmter Grenzen stufenlos winkelbeweglich gelagert un in allen Einstellagen arretiert. Die Halterung der Spanndrähte ist jedoch nur in Durchmesserrichtung möglich, das durch zwei Spanndrähte gebildete Kreuz ist nicht beliebig in der Ebene des Ringes verfahrbar.

Durch die DE-A-3510305 ist eine Vorrichtung zur externen Fixation von Knochensegmenten bekanntgeworden, die wenigstens zwei Ringe enthält, an denen sich kreuzende Kirschner-Drähte gespannt werden. Die Ringe werden mit über ihren Umfang verteilt angeordneten Stäben in einem einstellbaren Abstand gehalten. Zur Anbringung der Stäbe dienen stufenlos verstellbare Halter, die von den Ringen radial auswärts auskragen. Am Ende der Halter liegt ein dem Stab aufnehmendes Gelenk mit Drehfreiheit um wenigstens zwei zueinander senkrechte Achsen. Zwischen die Ringe ist an jedem Stab ein Federspanner mit einstellbarer, in Stablängsrichtung wirkender Federkraft eingeschaltet. Dieser Ringfixatuer besitzt im wesentlichen die gleichen Nachteile, wie sie beim Ringfixateur der DE-OS 34 39 795 beschrieben worden sind.

Durch die EP 0194187 A1 ist ein Ringfixateur bekanntgeworden, der aus Ringen mit kreisförmigem Querschnitt besteht, die miteinander durch längsverlaufende Streben verbunden sind. Die Streben bestitzen an beiden Enden je eine Halbkugel mit einer geriffelten Fläche mit der Normalen senkrecht in Richtung der Strebe, wobei dieser Fläche eine ähnlich gestaltete Halbkugel mit geriffelter Fläche gegenübersteht, die integrales Teil einer Bolzenhalterung ist, die am Ring klemmbar befestigbar ist. Durch beide Halbkugeln ist eine Schraube geschraubt zur reibschlüssigen Verbindung eines Endes des Stabes mit der Bolzenhalterung, so daß der Stab innerhalb der Bolzenhalterung um einen Freiheitsgrad beweglich ist. Die Bolzenhalterung sitzt mit ihrem anderen Ende in einer Schraubklemme, die entweder den Ring oder auch eine weitere Strebe in klemmender Weise zu umgreifen imstande ist.

Ähnlich wie die Halbschalen weisen die Schraubklemmen seitlich eine geriffelte Anlagefläche auf, die dazu dient, zwei Schraubklemmen mit einer langen Schraube gleichzeitig zu klemmen, wobei die beiden Schraubklemmen um einen Winkel von 360 Grad zueinander verdrehbar sind. Dadurch ist eine gewisse Parallelverschiebung der Ringe und der Streben zueinander möglich. Nachteilig ist jedoch, daß zum Verstellen der Streben zu den Ring-Halterungen, wie auch der Schraubklemmen untereinander die jeweils durchgehende Schraube vollständig gelöst werden muß, um aufgrund der Riffelung der aneinander pressenden Flächen die Winkelstellung zu ändern. Dadurch können sich die mit dem Ringfixateur aufgebauten Spannungsverhältnisse in unkontrollierbarer Weise ändern. Ebenso ist die Manövrierfähigkeit der einzelnen Teile zueinander zur beliebigen Verstellbarkeit des Ringfixateurs beschränkt; eine linienschlüssige Klemmbesfestigung in den Schwenklagern der Streben ist nicht gegeben.

Technische Aufgabe:

Der Erfindung liegt deshalb eine in drei Teilaufgaben zu untergliedernde Gesamtaufgabe zugrunde, einen verbesserten Ringfixateur der eingangs genannten Gattung auszubilden; es ergibt sich eine stereometrische, eine dynamometrische und eine taktische Teilaufgabe.

Die stereometrische Teilaufgabe verlangt die stufenlose Einstellbarkeit der Längen wie auch der Winkelstellungen der Streben gegenüber den Ring- und Teilringebenen, sowie die beliebige Ortswahl auf den Ringen für die Befestigung der Streben- und der Drahthalterungen und die beliebige Ortswahl für Drahtbefestigungen zwischen den Ringen und an den Streben selbst.

Die dynamometrische Teilaufgabe verlangt die Einstellmöglichkeit aller Spannlagen, die zwischen Ringfixateurteilen selbst aufzubringen sind und ebenso aller Spannlagen, die sich aus dem Verbund zwischen dem Ringfixateur einerseits und den Knochen- bzw. Weichteilgeweben andererseits ergeben derart, daß sie stufenlos und ohne Hinzunahme eines zusätzlichen Spanngerätes vorgenommen werden können.

Die taktische Teilaufgabe verlangt eine derartige Einfachheit der Bedienung, daß bei Ringfixateur-Montage zu Therapiebeginn wie auch während der gesamten therapeutischen Fixationsphase im Ringfixateur alle stereometrisch fixierenden und alle dynamometrisch spannenden Maßnahmen mit wenigen gleichartig wiederkehrenden Handbewegungen des Verstellens und des Verspannens an allen zu bewegenden und Kraft aufnehmenden Teilen ausführbar sind, wobei der ergänzende Einsatz, die Wegnahme oder der Austausch von Halterungen, Teilringen, Streben und Drähten je für sich und zwar in ring-radiärer Montagerichtung - also ohne Störung der übrigen Fixationsanordnung - ausführbar sein sollen.

Darstellung der Erfindung

Die Lösung dieser dreigliedrigen Aufgabe besteht erfindungsgemäß in den Merkmalen des Anspruchs 1. Weitere Ausgestaltungen der Erfindung sind in den Unteransprüchen gekennzeichnet.

Ein erster entscheidender Vorteil der erfindungsgemäßen Vorrichtung geht von den Drähten aus, welche zum Aufschrauben von Spann- und Positionsmuttern als Gewindedrähte mit sich durchgehend erstreckenden Gewinden ausgebildet sind. Hierdurch wird die Spannbarkeit der Drähte erreicht, ohne daß es gesondert anzusetzender äußerer Spannmittel bedarf. Zur Erfüllung des biomechanisch ausgelegten Therapiekonzeptes gilt für Ringfixateure nämlich, daß sie stereometrisch und dynamometrisch an die natürlichen Heilungsprozesse anpaßbar sein müssen; daß heißt, daß prozessual sowohl die Lage wie auch die Spannung jedes einzelnen Drahtes regulierbar zu sein hat. Da diese Korrekturen methodengemäß häufig in kleinen Schritten - meistens täglich - erfolgen müssen, bedeutet das einfache spannungsregulierende Drehen an den erfindungsmäßigen Drahtspannschrauben eine erstmals praktikable Umsetzung von prozessualen Osteosynthesetechniken an sich.

Vorteilhaft ist des weiteren die Gewindeausrüstung der Drähte deshalb, weil während der Drahteindrillung, die gegensinnig, also für Draht- Rechtsgewinde mit linksdrehender Drahtdrillung, ausgeführt werden kann, die Drahtgewinde den Rücktransport des entstehenden Knochenbohrmehls bewirken und weil auf diese Weise die Bohrlochwandungen durch nachfließendes Blut und Gewebswasser vor übermäßiger Bohrhitze geschützt werden.

Vorteilhaft sind die Gewindedrähte des weiteren, weil an jeder gewünschten Stelle der Drähte die erfindungsmäßig an der Oberfläche polyederförmig-kantig gestalteten, drehfest konterbaren Positionsmuttern - bei Ilisarov oberflächenglatt, mit den Drähten metallisch verbunden und als "Oliven" bezeichnet - aufgeschraubt werden können und weil somit die Bevorratung mit besonderen "Oliven-Drähten" entfallen kann.

Vorteilhaft ist schließlich die Gewindeausbildung auch deshalb, weil die Drähte über die sphärisch lagernden Spannmuttern in geschwenkte und dennoch knickungsfreie Spannlagen gebracht werden können.

Ein weiterer entscheidender Vorteil der erfindungsgemäßen Vorrichtung geht von den Drahtlagern zum Verbinden dieser Gewindedrähte mit den Ringen und Ringteilen oder den Streben dadurch aus, daß sie aus flächenschlüssig klemmbaren U-förmigen Reitern bestehen, die auf dem Umfang der Ringe oder auf den Streben in Ringradius-Richtung oder in einer hierzu um 90 Grad gedrehten Richtung aufsteck-, abnehm- und verriegelbar und in Verriegelung noch frei verschieblich aber auch klemmbar sind. Betrachtet man die erfindungsgemäße Vorrichtung aus anatomischer und operationsmechanischer Sicht, so besteht der Vorteil der beliebigen U-Reiter-Verschieblichkeit anstelle üblicher Gebundenheit von Drahthaltern an vorbestimmte Lagerorte darin, daß diese freie Ortswahl der Befestigung stets die Möglichkeit bietet, bei der Drahtbohrung anatomotopographisch gefahrvollen Drahtpenetrationsorten und -richtungen aus dem Weg gehen zu können.

Bei einer sekundären Maßnahme, wie Drahtaustausch und Neulegung eines Drahtes, ist ebenfalls eine fein abstimmbare Drahtplatzierung von Vorteil. Auch dynamometrisch hat die Wahlfreiheit der Drahtlagen einen bedeutenden Vorteil, denn es muß immer die Spannungslosigkeit der Drähte die Ausgangssituation einer fertiggestellten Ring-Draht-Fixateurmontage sein, wenn stereometrische und dynamometrische Manövrationen therapeutisch zielsichere und keine anderen Spannlasten herbeiführen und auf die Knochenfragmentkonstellation übertragen sollen.

So ist es auch durch diese stufenlose Verschieblichkeit der U-Reiter in vorteilhafter Weise möglich, bereits eingebohrte Drähte durch nachträgliches Verrücken der U-Reiter gezielt zu verbiegen, um von solchen Verbiegungen ausgehend durch Erhöhung der Drahtspannung die Drahtbegradigung herbeizuführen, unter welcher der transfixierte Knochenabschnitt quer zur Drahtrichtung beigezügelt werden kann. Formgebung und Kraftflußveränderungen werden auf diese Weise räumlich exakt einstellbar bzw. dosierbar auf heranwachsende und sich beanspruchungsspezifisch organisierende Knochen- und Bindegewebsregenerate übertragbar.

Vorteilhaft ist an diesen erfindungsmäßigen Drahtlagerungen weiterhin, daß gebogene oder unter Lastaufnahme sich verbiegende oder unter Spannungszunahme ihr Ausbiegungsmaß ändernde Drähte nicht knicken, da sie durch lösbar mit den U-Reitern verbundene, sphärische Stützflächen aufweisende Spannlager von wählbarer Länge gesteckt und darin dreidimensional verschwenkbar gelagert werden können. Diese schwenkbare Lagerungsfähigkeit der Gewindedrähte und ihre Spannbarkeit in knickungsfrei geschwenkter Position ist erst durch die erfindungsgemäße Ausbildung von Spannmuttern mit sphärischen Lägerflächen möglich. Vorteilhafterweise können diese Kugelkopfspannmuttern außerdem auch mit ihrem Kugelkopf nach außen gewendet als Verletzungsschutz dienen. Dagegen ist es bei den herkömmlichen Drähten notwendig, die Enden einzubiegen, wodurch die Drähte für ein Nachspannen praktisch unbrauchbar werden.

Vorteilhaft ist auch, daß diese Lochlager um ihre Längsachse drehbar, in jeder Drehstellung befestigbar und austauschbar sind. So können je nach Bedarf kürzere gegen längere steckbare Lochlager ausgetauscht werden und umgekehrt. Bei erforderlichem Drahtaustausch und Mitaustausch der Lochlager kann die von einer aufzugebenden Knochenperforation gezielt Abstand nehmende neue Drahtbohrung wiederum an anatomisch unbedenklichem Ort vorgenommen werden.

Stets vorteilhaft ist an den schwenkbaren Lochlagern, daß bohrlehrenartig wirkende Hülsenstücke eingeschoben werden können und bei der Drahtbohrung derart die Bohrrichtung festlegen, daß jeder Draht, in präziser Parallelität zur Ringebene verlaufend, das gegenüberliegende Lochlager erreichen kann, ohne eine korrigierende Verbiegung zu erleiden. Werden an einem Draht lediglich die vorhandenen Lochlager bei gleicher U-Reiter-Lage gegen Lochlager anderer Länge ausgetauscht, so kann eine gezielte Drahtausbiegung in Richtung der Ringfixateurhöhe bzw. der Knochenlängsachse erreicht werden, was therapeutisch durch drahtbegradigende Drahtspannungserhöhung sowohl zur Höhenlenkung als auch zur Beeinflussung der Lagestabilität derart transfixierter Knochenabschnitte genutzt werden kann.

Ein weiterer wichtiger Vorteil der erfindungsgemäßen Drahtlagerungen liegt in ihrer U-förmigen Symmetrie. Bei Bedarf kann in ein- und denselben U-Reiter zu beiden Seiten eines Ringes bzw. einer Strebe je ein Draht eingespannt werden. Die damit auch für die Spannkräfte gegebene Symmetrielage bewirkt, daß die von den Drähten ausgehenden entgegengesetzt gerichteten Drehmomente sich neutralisieren, sodaß demzufolge unerwünschte Verformungen am Ringfixateurgerüst vermieden und hohe therapeutisch nutzbare Spannlasten aufgebracht werden können.

Ein maßgeblicher Vorteil ist auch die Verwendbarkeit der U-Reiter für die Befestigung der erfindungsmäßigen Strebenlager. Zum Zusammenfügen ist es lediglich nötig, steckbare Kupplungsglieder einzusetzen, die mit entsprechend geformten Enden in die Steckbuchsen der U-Reiter und in die Klemmhalterungen der Strebenlager gesteckt werden. Diese Art der Strebenbefestigung liefert in vorteilhafter Weise die Möglichkeit, an ein- und demselben U-Reiter entweder mittels eines oder zweier Lochlager ein oder zwei Drähte oder mittels eines oder zweier Kupplungsglieder ein bis zwei Strebenlager oder in gemischter kombination jeweils ein Drahtlager und ein Strebenlager über den U-Reiter an einem Ringort zu befestigen.

Die Vorteile dieser Kombinierbarkeit liegen zum einen in der sich dadurch vergrößernden Montagevielfalt bei gleichzeitiger Einsparung von Montageorten je Ring, zum anderen in einer erheblichen Gewichtseinsparung dank der sich reduzierenden Zahl von U-Reitern. Die Strebenbefestigung mittels der U-Reiter ermöglicht, daß jeder Ort des Ringumfanges als ein Ort für eine Strebenlagerung ausgewählt werden kann. Dies ist insoweit notwendig, als immer zuerst den Befestigungsmitteln der Drähte die bestmögliche Platzierung gebührt, währenddessen die Wahl der Streben-Befestigungsorte ausweichend und in Anpassung an die Drähte-Priorität erfolgt.

Ein weiterer Vorteil ist, daß eine vollständige, den Ringverbund destabilisierende Strebendemontage zur Ausführung ausweichender Streben-Querverschiebungen nicht notwendig ist, sondern lediglich eine reibschlüssige Lockerung der Klemmspannung der U-Reiter auf dem Ring.

Vorteilhaft für das einfache, zumeist der prozessualen Längenanpassung dienende Anbringen und Austauschen einzelner oder aller Streben ist, daß die Streben samt der U-Reiter in ring-radiärer Montage-Richtung an den Ringen anbringbar und in entgegengesetzter Richtung von denselben abnehmbar sind. Da also durchstoßende Montagen zwischen Ringen und Streben erfindungsgemäß überall vermieden werden, sind die ummontierenden Manipulationen am Ringfixateur einfach, und es können Stabilitätsrisiken und unbeabsichtigte Knochenfragment-Mitbewegungen auch in interkurrenten, prozessual im Therapieverlauf notwendig werdenden Umbauphasen sicher vermieden werden.

Weiterhin von entscheidendem Vorteil ist die erfindungsmäßige Ausgestaltung der Streben mit in der Strebenachse angeordneten spaltförmigen Ausnehmungen und mit austauschbaren, spindelförmigen Abschnitten zu ihrer Anpassung an in ihrem Abstand veränderliche Ringetagen.Diese Ausnehmungen können Längsschlitze zwischen zwei Teilstreben oder eine Reihe von in der Mittellinie der Streben angeordneten Perforationen sein, durch welche daselbst lager- und schwenkbare Fixations- und Lenkdrähte gesteckt werden können.

Wesentlicher stereometrischer Vorteil sind die dadurch orts- und richtungsmäßig frei bestimmbaren Drahtlagen im Zwischenbereich zwischen den Ringen, die durch das Zusammenwirken der Strebenschlitze, der drehbaren spindelförmigen Strebenabschnitte und durch die schwenkbar lagernden Drahtspannmuttern ermöglicht werden.

Ein wichtiger Vorteil der Strebenlängsspaltung ist auch darin zu sehen, daß die Durchstoßpunkte der drahtspannenden Kräfte auf der Mittelachse der Streben liegen, sodaß es nicht unter Drahtanspannung zu Drehmomenten an den Streben und somit auch nicht zu unerwünschten Mitbewegungen der an diesen Drähten fixierten Knochenfragmenten kommt.

Die austauschbaren spindelförmigen Strebenabschnitte bringen neben der axialen Drehbarkeit und der spannzangenmäßigen Klemmbarkeit der Spindeln auch den Vorteil, daß im Zusammenwirken mit den noch zu beschreibenden Längslagern der Streben durch Spindelaustausch grobe und durch teleskopische Schraubungen feine Längenanpassungen der Streben vorgenommen werden können.

Von entscheidendem Vorteil und notwendig für die Erfüllung aller Teilforderungen der technischen Aufgabe ist, daß die Strebenlager zum Verbinden der Streben mit den Ringen und/oder Ringabschnitten Spannhülsen zum flächenschlüssigen Klemmen und stufenlosen Verstellen aufweisen, die sowohl längen- als auch dreidimensional winkelanpassende Einstellungen der Streben ermöglichen, wobei die Einstellungen für lineare Freiheitsgrade von Einstellungen für rotatorische Freiheitsgrade getrennt ausführbar sind. Der flächenmäßige, gegenüber Schubkräften rutschsicher zu machende Klemmschluß wird jeweils durch spannzangenartige Schlitzungen der Spannhülsen unter Ausbildung von Halbschalen erreicht, und zwar sowohl am längenanpassenden Achslager wie auch am winkelanpassenden Kugellager. Zugschrauben, die in fluchtenden Flanschbohrungen der Spannhülsen gelagert sind, erbringen mit vorteilhaftem ring-radiärem Zugangsweg die erforderlichen Klemmspannungen.

Besonders vorteilhaft ist, daß die gelagerten Gelenkkugeln unterschiedlich gehandhabt werden können, und zwar je nach eingebrachter Spannkraft der Zugschrauben austauschbar, oder frei beweglich jedoch vor Ausrenkung gesichert, oder durch höhere Reibungskräfte gezielt schwergängig, oder durch hohe Flächenpressung ganz rutschfest.

Die klemmbaren Achslager der Spannhülsen haben den besonderen Vorteil, daß zur Verdrehsicherung und axialen Stabilisierung der Spindeln keine Kontermuttern in aufwendigen Arbeitsgängen bedient werden müssen, sondern daß ebenso wie am benachbarten Kugellager über Zugschraubenkräfte stabile Klemmspannungen aufgebracht werden können. Günstig ist an der spannzangenartigen Gewindeklemmung weiterhin, daß auch bei kurzstreckiger Einlagerung von Spindeln wackelfreie Passung erreicht werden kann.

Von Vorteil ist auch, daß vermöge solcher spannzangenartigen Lagerunterteilung auch im Falle konzentrisch zwischengelagerter Hülsen die nötigen Klemmkräfte auf eine zentrale Achse hindurchgreifen können, wenn wie im Falle der Schraubhülsen dieselben erfindungsmäßig mit Längsschlitzen versehen sind.

Ein ebenfalls entscheidender Vorteil des erfindungsgemäßen Ringfixateurs ist die Ausbildung der Ringe und Ringteile in der Form, daß sie im Wandquerschnitt quadratisch oder hochformatig-rechteckig und aus in Umfangsrichtung endlos unidirektional gewickelten, in Matrix gebetteten Fasern ausgebildet sind.

Bei derartiger Ringgestaltung als Zylindersegmente ist es in vorteilhafter Weise möglich, die Wandstärke gering zu halten; und zwar dann, wenn erstens ein Faser-Matrix-Materialverbund verwendet wird, welcher durch die Konstellation aus zugfesten Fasern und druckfestem Bettungsmaterial hohe elastische Rückstellkräfte ohne plastische Verformungsrückstände besitzt, und wenn zweitens eine solche Zylinderhöhe gewählt wird, daß Torsionsstabilität gegeben ist. Dabei kann in vorteilhafter Weise genutzt werden, daß die Torsionsstabilität eines Ringes der dritten Potenz der Höhe dieses Ringes proportional ist. So können diese Ringe relativ niedrig und dünnwandig ausgebildet sein, ohne Stabilitätseinbußen zu erleiden.

Ein weiterer Vorteil dieser Ring- und Teilringgestaltung ist, daß sie den Gliedmaßen nahe anliegen, also im Durchmesser klein gehalten werden können. Sie sind also im Gegensatz zu herkömmlichen Ringfixateuren mit flächig geformten, großen Durchmesser erfordenden Ringen platzsparend und für die Kranken weit weniger hinderlich. Die Patienten können die Ringfixateure dank solcher Ringe ohne weiteres in vorteilhafter Weise auch unter Hosenbeinen und Ärmeln tragen.

Die erfindungsgemäße Wahl eines quadratischen Querschnitts hat den Vorteil, daß die anzuklemmenden U-Reiter auch in einer um 90 Grad gedrehten Stellung befestigbar sind, was eine erhebliche Erweiterung der stereometrischen Montagevielfalt bedeutet.

Auch ist von Vorteil, daß Teilringe zu Vollringen über ein stabilisierendes Steckschloß zusammengefügt werden können. Dabei erweist es sich als günstig, daß an einem derartigen, die Ringteile zum ganzen Ring verbindenden Schloß wiederum mit Platzeinsparung an der Ringzirkumferenz und unter Gewichtsreduktion Gewindedrähte und/oder Streben mitbefestigt werden können.

Eine vorteilhafte weitere Ausgestaltung ist in der Ausbildung von Schraubkappen gegeben, die zum Schutz vor Verletzung auf die Drahtenden aufgebracht, zum Ansatz eines drahtbohrenden Spannfutters abgeschraubt, zum Bedienen der Spannmuttern jedoch belassen werden können. Vorteilhaft ist dabei, daß die Drähte nicht zum Schutz vor Verletzung umgebogen werden müssen. Sie bleiben dadurch auch zur Wiederverwendung intakt.

Eine weitere vorteilhafte Ausgestaltung besteht in der Ausbildung von flanschtragenden Spindeln mit Gewindeabschnitt, mit Schlüsselflächen und/oder Lochkranz am Flansch und mit endständiger Ringnut, da nacheinander in der Reihenfolge des Steckens, Verriegelns und drehfesten Verklemmens diese Spindeln mit den spaltförmigen Strebenabschnitten zusammengefügt werden könnnen, sodaß durch teleskopisches Zusammenwirken solchermaßen spindelförmig ergänzter Streben mit den vorerwähnten hülsenförmigen Strebenlagern die Längenanpassungen an die zu überbrückenden Ringebenen-Abstände erfolgen können. Nachdem zunächst zur Ausführung dieser Längenjustierungen die ankoppelnde Zapfenlagerung zur Verbindung der Strebenabschnitte mit Andrehen der in die Ringnut eingreifenden Stellschraube benutzt und bedarfsweise die drehfest sichernde Spindelklemmung erzeugt wurde, wird es sodann möglich, schraubtätige Längeneinstellungen entweder durch Drehung des Strebenlagers unter Entspannung beider Spannzangenlager oder durch Drehung der Spindel unter Entspannung ihrer beiden endständigen Achslager vorzunehmen.

Ein zusätzlicher Vorteil solch austauschbarer spindeliger Strebenenden ist auch darin zu sehen, daß das Sortiment an verfügbaren, längsperforierten bzw. längsgeschlitzten Strebenabschnitten verringert werden kann, da die grobe Längenanpassung der Streben über ein Sortiment verschieden langer Spindeln regelbar ist.

Vorteilhaft vor allem für das Handling sind Verriegelungsstecker zu formschlüssiger Klemmspannung zwischen U-Reitern einerseits und den Ringen bzw. den Streben andererseits. Diese Steckriegel verschließen die U-Öffnungen der Reiter und übernehmen mittels der Zapfen räumlich schlüssige Paßspannungen für wackelfreien U-Reiter-Sitz.

Ein Dislozieren der Stecker wird durch Spaltung der Zapfenenden erreicht, die in Steckposition bei geringfügig divergierenden Hälften einen splintartigen Reibschluß als Schutz vor Herausfallen erzielen. Diese Schlitzung wirkt vorteilhaft, weil ein einfach zu erbringender und ebenso leicht veränderbarer klemmschlüssiger Sitz des Steckriegels erreicht werden kann.

Eine weitere vor allem dynamometrisch vorteilhafte Ausgestaltung der Erfindung ergibt sich bei der Ausbildung von Gewindespindeln, die paraaxial an den Streben befestigbar sind, und auf denen Stellmuttern aufschraubbar sind, durch deren Verdrehen die Höhenstellung der Drahtlager bestimmt werden kann. Diese im Behandlungsprozeß vorzunehmenden Höhenverstellungen der interetagär liegenden Drähte erzeugen eine Ausbiegungs- und Spannungsänderung an in benachbarten Etagen liegenden Drähten ein- und desselben Knochenfragmentes. Man erreicht auf diese Weise eine fein dosierbare Einstellung der Lagestabilität der Knochenfragmente und kann angepaßt an den phasenhaften Ablauf der autoregulativen natürlichen Ossifiktions- und Knochentransformationsprozesse gezielte Drahtspannungseinstellungen vornehmen, sowie in Adaptation an die natürlichen Heilungsvorgänge stetige Regulierungen des Spannungsmaßes.

Sehr vorteilhaft ist, daß sich dank einer solchen erfindungsmäßigen, spindelgelenkten Transportiervorrichtung drahtgezügelte Knochenfragmentversetzungen als sogenannte Knochenverschiebeplastiken aus -führen lassen.

Als besonderer Vorteil der als Rahmen aus zwei längsachsen-parallelen Trägern und zwei dazu rechtwinkelig gestellten Rahmenschenkeln gebildeten Streben ist anzusehen, daß sich die unter Zugspannung stehenden Spannmuttern an den glatten,spaltbildenden Wandungen der Träger abstützen können, ohne in Spannungsrichtung durchzurutschen und ohne daß besonders halternde Lochlager notwendig sind, und daß sie bei einem therapeutischen Längenänderungsprozeß einer Gliedmaße in Zugrichtung, verbunden mit dem an ihnen gehalterten Draht, mitwandern können, ohne daß sich die Drahtspannung erhöht, währenddessen sie dennoch zur Neutralisierung gefährlicher Fragmentausbiegungen zur Seite und gegenläufiger Fragmenttorquierungen beitragen.

Eine vorteilhafte größere räumliche Montagefreiheit ergibt sich im Bedarfsfalle - insbesondere bei doppelläufigen Knochenkonstellationen - wenn bei Einbringung von Gewindedrähten im interetagären Bereich zwischen zwei Ringen für deren Lagerung zapfengelenkig aneinandergekoppelte Rahmenabschnitte zu Streben zusammengesetzt werden. Zur Verbindung dieser Strebenteilabschnitte eignen sich gemäß einer bevorzugten Ausführung zylindrische Kupplungs achsen, die durch an beiden Zylinderenden umlaufende Ringnuten verriegel- und klemmbar sind. Mit ihrer Hilfe können solche rahmenförmige, mit entsprechenden Zapfenlagern ausgestattete Strebenrahmen zusammengesetzt, gegensinnig verdreht und auch gegeneinander drehfest verriegelt werden. Dadurch wird es möglich, Gewindedrähte, von einer Strebe ausgehend, in wählbarem Höhenabstand und in verschiedene horizontale Richtungen weisend zu bohren und, von derartig divergenten Lagen ausgehend, von einander unabhängig zu verschwenken.

Vom erfindungsgemäß vereinfachten Handling hervorgerufen, ergeben sich besondere Anwendungsvorteile dieses Ringfixateurs:

Die Einfachheit der Erstmontagen und jeden Um-Montierens, die auf der Steckbauweise dieses Ringfixateurs beruht, ermöglicht eine beschleunigte Operationsausführung, sodaß auch in schwierigen Osteosynthesefällen die ambulante Durchführung - möglicherweise sogar in örtlicher Betäubung - in Betracht gezogen werden kann. Dies bedeutet erfindungsbedingt, daß sich Anwendungsmöglichkeiten ergeben, die sich für Ringfixateure sonst nicht ohne weiteres auftun, aber ihr eigentliches Einsatzfeld darstellen könnten: nämlich der wehrmedizinische Einsatz bereits in vorderer Linie auf Hauptverbandplatz-Niveau, des weiteren der Großeinsatz bei Katastrophenlagen und die Verkehrs-Massenunfälle, jeweils mit ungenau kalkulierbarem Andrang von vielen und Vielfachverletzungen, sowie Schwerstverletzungen, bei denen die lebensrettenden Therapieeinsätze weitgehend unter den Aspekten versorgungstaktischer Triage-Richtlinien erfolgen und zeitaufwendige und blutvergießende Knochenoperationen wegen des überlebensrisikos unmöglich sind. Je rascher und je weniger weichteil- und knochentraumatisierend eine Operationsmethode als versatile Gewindedrähte zur Fixation verwendende Ringfixation ausgeführt werden kann, desto geringer ist das Infektionsrisiko und desto weniger kommen Blutkonserven zum Einsatz, die immer die Gefahr der Krankheitsübertragung beinhalten.

Schließlich ergibt sich aus der dargestellten bevorzugten Ausgestaltung des erfindungsgemäßen Ringfixateurs ein wesentlicher Produktionsvorteil: die meisten Bauteile sind, veranlaßt durch die Forderung nach ungehinderter dreidimensionaler Manövrierfähigkeit, als Drehteile konzipiert und auf computergesteuerten Drehautomaten herstellbar, sodaß die Variationen der Ausgestaltung, die sich innerhalb des Rahmens einer Erfindung in der praktischen Erprobungsphase ergeben, durch Programmänderung der Drehautomaten und unter Verzicht auf den kostspieligen Formenbau realisierbar sind.

## Kurzbeschreibung der Zeichnungen

Beispiele der Erfindung sind in den Zeichnungen dargestellt und anschließend beschrieben. Dabei zeigen:

Figur 1 eine Draufsicht auf einen Ringfixateur-Ring unter Darstellung von zwei sich kreuzenden Drähten und von den sie am Ring halternden Drahtlagern

Figur 2 einen Längsschnitt durch eine auf einen Gewindedraht aufgeschraubte Draht-Spannmutter

Figur 3 eine zentripetal in Drahterstreckung gerichtete Draufsicht auf eine Draht-Spannmutter

Figur 4 eine Längsansicht eines Paares von auf einen Gewindedraht in zufälliger Drehstellung gegeneinander gekonterten polyederförmigen Positionsmuttern

Figur 5 eine in Drahterstreckung gerichtete Draufsicht auf diese Positionsmuttern in gegenseitiger Drehstellung wie Fig.4

Figur 6 eine frontale Draufsicht eines auf einen Ringabschnitt aufgesetzten Drahtlagers mit einem Lochlager und mit einem Kupplungsglied, die auf dem Ring-U-Reiter des Drahtlagers gehaltert sind

Figur 7 einen Vertikalschnitt durch einen Drahthalter mit Lochlager und Kupplungsglied in Verriegelungsposition, wobei zur übersichtlichkeit alle Schnittschraffuren weggelassen sind

Figur 8 eine Seitansicht eines auf einen angeschnittenen Ring aufgesetzten Ring-U-Halters, wobei der Wandquerschnitt des Ringes quadratisch ist

Figur 9 eine frontale Ansicht einer Strebe, aufgebaut aus einem Rahmenabschnitt mit zwei einen Längsspalt einrahmenden Trägern, aus zwei an diesem Rahmenabschnitt angelagerten Schraubhülsen, die sich jeweils in ein Strebenlager hineinerstrecken, sowie jeweils aus einem Kupplungsglied, das kugelgelenkige Verbindung zu den beidseitigen Ring-U-Haltern herstellt

Figur 10 eine Seitansicht einer gemäß Fig.9 an zwei Ringe angesetzten Strebe, wobei die Ringe angeschnitten sind

Figur 11 eine frontale Ansicht eines Rixateur-Ringes Mit drei Drahtlagern in Frontal- bzw. in Seitansicht bzw, in einem median-vertikalen Schnitt

Figur 12 eine Seitansicht eines Ring-Verriegelungssteckers

Figur 13 eine Draufsicht auf einen in einen Ring-U-Halter gesteckten Verriegelungsstecker

Figur 14 einen Median-Vertikalschnitt durch eine Strebenlager-Spannhülse mit einem Kugel- und einem Hülsenlager, die voneinander beabstandet angeordnet sind

Figur 15 einen Horizontalschnitt (A-B in Fig.14) durch das Hülsenlager einer Spannhülse mit Darstellung der diametralen Wandschlitzung und der paarigen Spannschraubenlager

Figur 16 einen Horizontalschnitt (C-D in Fig.14) durch das Kugellager einer Spannhülse mit Darstellung der diametralen Wandschlitzung und der paarigen Spannschraubenlager

Figur 17 eine Längsansicht einer Schraubhülse mit Außengewinde, Längsschlitzen (nur ein Schlitz ist dargestellt), Flansch und Lochkranz für Steckschlüssel

Figur 18 einen Längsschnitt einer Gewindespindel mit Steckloch-Kranz im Flansch und mit einem eine Ringnut aufweisenden steckbaren Ende, das in Verriegelungsstellung im Rahmenquerbalken gelagert ist

Figuren 19 - 22 verschiedene Ansichten von einer paraaxial an die Strebenrahmen angelagerten Tranportiervorrichtung der Streben-Drahtlager mit Gewindespindel und dem Verbund von Stellmutter und klemmbarem, draht-führendem Streben-Drahtlager zur Höheneinstellung der Drahtlagen

Figuren 23 - 26 verschiedene Ansichten und Schnitte der zusammensteck-, verriegel-, verdreh- und feststellbaren Verbindung von zwei rahmenförmigen Strebenabschnitten, die mittels einer Kupplungsachse hergestellt wird

Figuren 27 - 31 verschiedene Ansichten und Ausgestaltungen von Lochlagern und Kupplungsgliedern und ein Halterungsbeispiel in einem median-vertikal geschnittenen Ring-U-Halter

Figur 32 Eine handgezeichnete räumliche Darstellung eines am Unterschenkel angelegten Ringfixateurs zur Fixation eines gebrochenen Schienbeins.

## Bevorzugute Ausführung der Erfindung

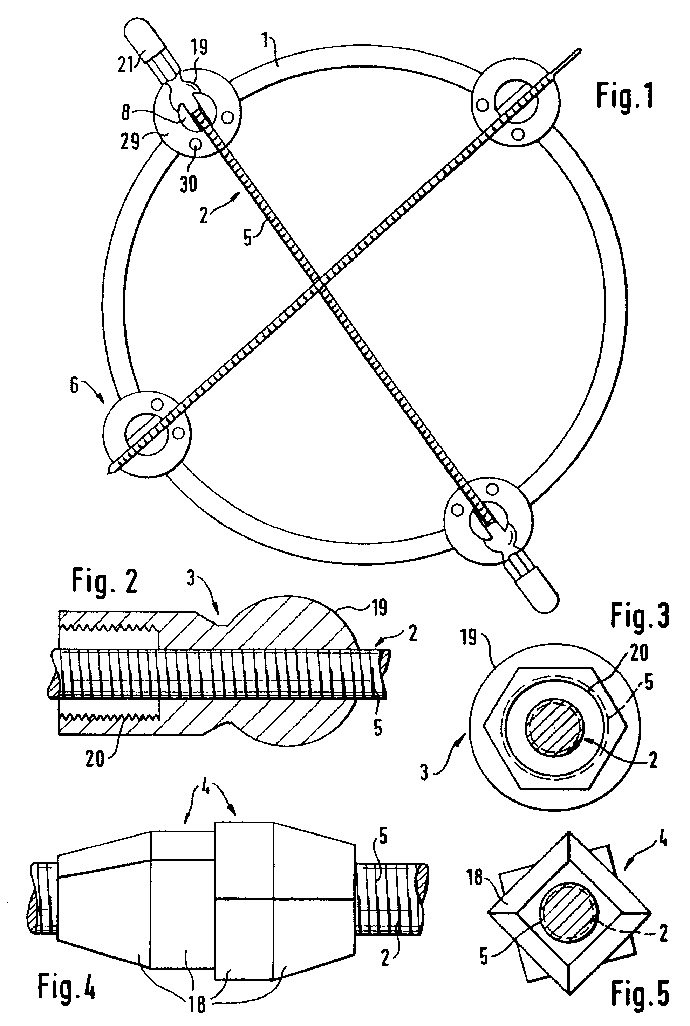

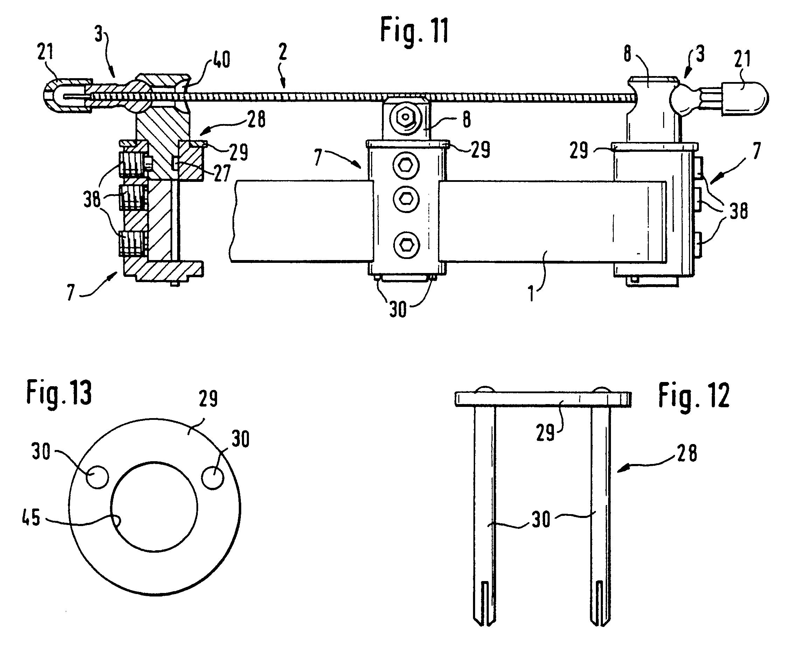

In den Figuren sind gleiche Teile mit gleichen Bezugszeichen gekennzeichnet. In Figur 1 und Figur 11 werden Draufsicht und frontale Ansicht eines Ringfixateur-Ringes 1 gezeigt. Auf ihm ist die Halterung von Draht-Lagern 6 veranschaulicht, die jeweils aus einem Ring-U-Halter 7 und einem Lochlager 8 - oder bei zur Ebene der Ringe 1 symmetrischem Aufbau aus zwei sich gegenüberstehenden Lochlagern 8 - bestehen. Die Anbringung der Ring-U-Halter 7 am Fixateur-Ring 1 geht so vor sich, daß der Ring-U-Halter 7, mit seiner offenen U-Seite zum Ring 1 weisend, wie im median-vertikalen Schnitt durch den linken U-Halter 7 der Figur 11 dargestellt, formschlüssig auf den Ring 1 an einem vom gesamten Montage-Plan her definierten oder aber an einem beliebigen Ort aufgesteckt und danach durch Einstecken eines Verriegelungssteckers 28 gesperrt wird.

Dieser Verriegelungsstecker, in den Figuren 12 und 13 in Seitansicht und Draufsicht dargestellt, besteht aus einer Tragplatte 29 und aus in ihr befestigten paarigen Zapfen 30, die den Ring 1 überragen und mit ihren freien Enden innerhalb des Ring-U-Halters 7 stecken. Nach Ausführung dieser Steckmontage ist der Draht-Halter 6 zunächst auf dem Ringumfang frei verschieblich. Der Verriegelungsstecker 28 fällt nicht aus den ihn aufnehmenden fluchtenden Bohrungen der U-Schenkel der Ring-U-Halter heraus, da die Enden der Zapfen 30 Schlitzungen aufweisen, die zum Zwecke eines genügend klemmschlüssigen Paß-Sitzes des Verriegelungssteckers 28 aufbiegbar sind. Nach der U-Halter-Verriegelung folgt dessen Verklemmung am Ring 1 derart, daß in die Basis des U's des Ring-U-Halters 7 einschraubbare Madenschrauben 38 bis zu einem am Ring kraftaufnehmenden Paß-Sitz hinzugedreht werden. Auf diese Weise entsteht infolge eines geringen Auswärtsrückens des Ring-U-Halters 7 eine einen stabilen Klemmsitz erzeugende doppelt linienförmige Kraftschlüssigkeit zwischen den Zapfen 30 und der Innenseite der Ringe 1. Dieser Klemmsitz kann mittels der Madenschrauben 38 einfach ohne weitere Demontage-Schritte gelockert und nach U-Halter-Verschiebung wieder hergestellt werden.

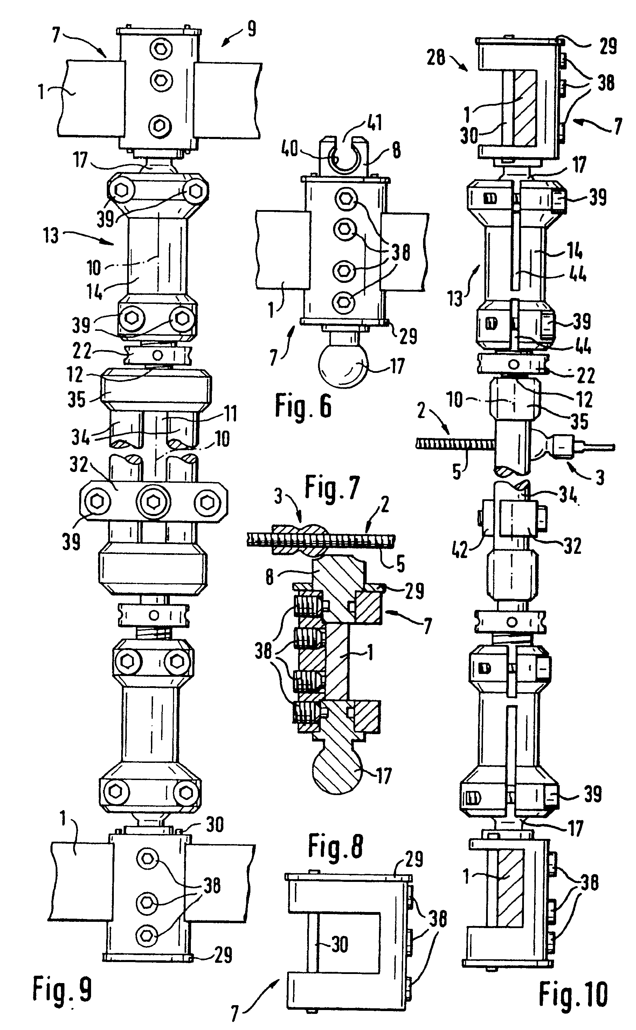

In einem Schenkel der Ring-U-Halter 7, wie in den Figuren 9, 10 und 11 dargestellt, oder aber in beiden Schenkeln, wie mit den Figuren 6 und 7 veranschaulicht, werden Lochlager 8 und/oder Kupplungsglieder 17 steck- und mittels Madenschrauben 38 verriegelbar gehaltert. Sie können jeweils durch entsprechend tiefe Eindrehung der Madenschrauben 38 in die vorgesehene Ringnute 27 ver- und zu Entnahme und-/oder Austausch entriegelt werden, sowie verdrehbar gehaltert oder in bestimmbarer Drehstellung drehfest arretiert werden. Bei dem Steckvorgang der Lochlager 8 und der Kupplungsglieder 17 gewährleistet die zentrale Perforation 45 der Tragplatte 29 des Verriegelungssteckers 28 den ungehinderten Durchtritt der in die Ring-U-Halter 7 zu steckenden Bauteile. Auch bei Gestaltung eines Ring-U-Halters 7 mit anhängender Kugelkopfkupplung (formgleich mit dem Kugelkopf des Kupplungsgliedes 17), wie in Figur 10 dargestellt, ist die Aufsteckbarkeit der VerriegelungssteckerTragplatte 29 gegeben.

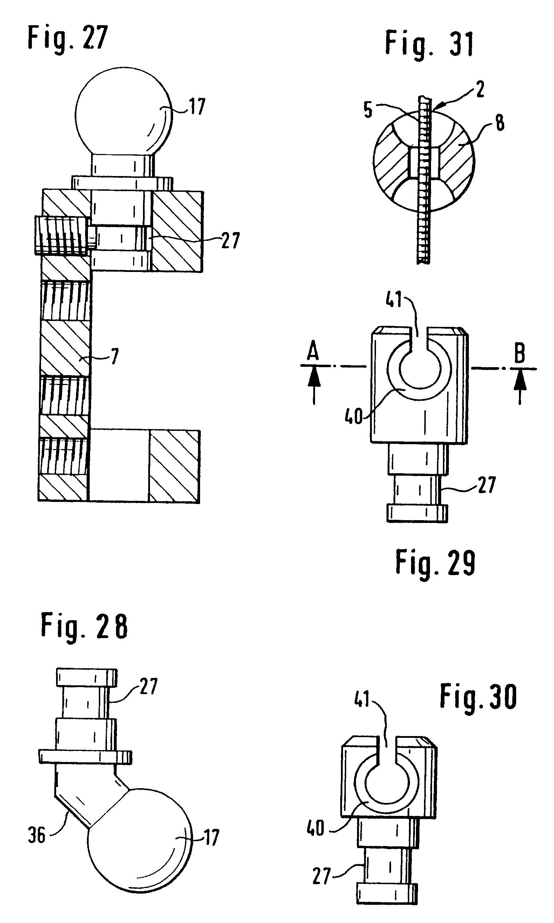

Die Figuren 29, 30 und 31 bringen Ausgestaltungen der Lochlager 8 zur Darstellung. Das Lochlager 8 besteht aus einem Zylinderteil, an welches sich ein verjüngtes Teil, ebenfalls in zylindrischer Form, anschließt, welches an seinem unteren Ende eine Ringnut 27 aufweist. Der Abstand zwischen Ringnut 27 und Lagerschale 40 des Lochlagers 8 kann verschieden sein, womit bei entsprechender Lochlager-Wahl der erforderliche Höhenabstand der Gewindedrähte 2 vom zugehörigen Ring wählbar ist. In symmetrischer Weise können sowohl an der Eintrittsstelle der Gewindedrähte 2 wie auch an ihrer Austrittsstelle Kugelschalen 40 in die Lochlager 8 eingesenkt sein, was der Montage-Vereinfachung als beliebige Verdrehbarkeit der Lochlager 8 zugute kommt.

Die Figuren 2 und 3 zeigen in Längsschnitt bzw. Draufsicht eine Draht-Spannmutter 3, die auf einem Gewindedraht 2 aufgeschraubt ist. Die Drahtspannmutter 3 hat eine sphärische Lagerfläche 19, mit der sie in der Lagerschale 40 des Lochlagers 8 verschwenkbar gelagert wird, wie dies aus Figur 31 hervorgeht. Die Gewindedrähte 2 sind über die Einschlitzungen 41 der Lochlager 8 auch von deren Stirnseite her in die Lagerschalen 40 einlegbar und entfernbar. Das Zusammenwirken der verschiebbaren Ring-U-Halter 7 mit den drehbaren Lochlagern 8 und den schwenkbar gelagerten Drahtspannmuttern 3 erbringt eine räumlich weitgehend unbegrenzte Einstellbarkeit der Gewindedrähte 2 bezüglich ihrer anatomisch orientierten Platzierung und ihrer mit Rücksicht auf verletzliche Nerven- und Gefäßstrukturen sorgfältig zu selektierende Penetrationsrichtung. Die Drahtspannmuttern 3 sind an ihren äußeren Enden innenwandig mit einem Gewindeabschnitt 20 für das Anbringen von derartig im Durchmesser begrenzten Schutzkappen 21 versehen, daß sie in Sechskant-Hohlschlüssel, die an den Sechskantflächen der Draht-Spannmuttern 3 angreifen, eintauchen können. Alternativ können die Schutzkappen 21, wie in Figur 1 dargestellt, auch bei größerer Dimensionierung auf die Draht-Spannmuttern formschlüssig aufgestülpt werden, wobei Randschlitzungen einen dosierbaren Klemmsitz solcher Schutzkappen 21 erzielen. Anstelle von gesondert gestalteten Schutzkappen 21 kann auch jeweils eine weitere Drahtspannmutter 3 seitenverkehrt auf den Gewindedraht 2 aufgeschraubt und an der schon vorhandenen Draht-Spannmutter 3 gekontert werden, sodaß die runde Spannmutter-Lagerfläche, nun außenseitig liegend, als Schutz vor Verletzung an den Drahtenden zur Geltung kommt.

Die Figuren 4 und 5 zeigen in Längsansicht bzw. Draufsicht gegeneinander stirnseitig konterbare, an der Oberfläche längsgerichtete Polyeder-Kanten aufweisende Drahtpositionsmuttern 4. Diese sind an jedem Ort der Gewindedrähte 2 durch gegenseitige Konterung drehfest befestigbar. Sie dienen längsgerichteter Drahtzügelung, wobei die stirnseitig konvergierenden Polyeder-Kanten kraftschlüssigen Kontakt mit den Knocheneintrittsorten der Gewindedrähte 2 aufnehmen und dieselben damit während eines drahtanspannenden Schraubvorganges in verdrehsicherer Position halten.

Die Figuren 7, 8 und 11 stellen u.a. dar, daß die Querschnitte der Ringwandungen hochformatig oder quadratisch sein können, sodaß neben der bevorzugten horizontalen, in Ringradius-Richtung auszuführenden Montage der Ring-U-Halter 7, wie sich dies bei Hochformat der Ringwand-Querschnitte ergibt, auch eine um 90 Grad gedrehte, vertikal zur Ringebene von oben oder von unten her kommende Steckung wählbar ist. Der Quadratische Ringwandquerschnitt induziert konsequente Ausgestaltungen der Ring-U-Halter bezüglich der Anschlußzurichtungen von befestigbaren Lochlagern 8 und Kupplungsgliedern 17.

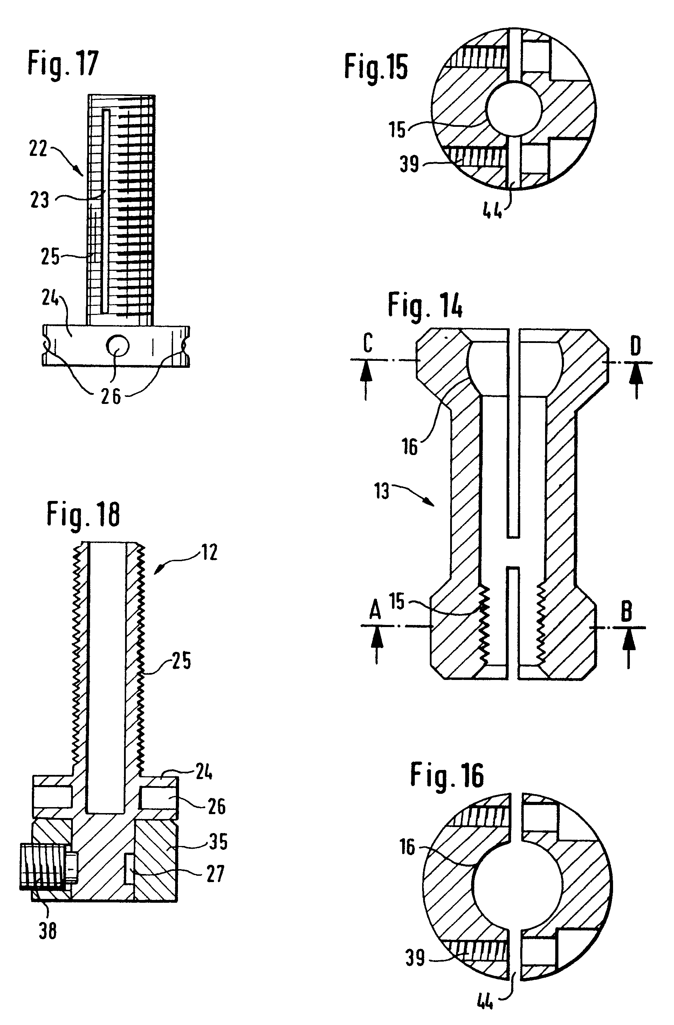

Aus den Figuren 9 und 10 gehen die bevorzugten Gestaltungen der verschiedenen Abschnitte der Streben 9 hervor. Mittels Ring-U-Halter 7 und Kupplungsglied 17 sind die Streben 9 zwischen den Ringen 1 in den Kugellagern 16 schwenkbar und mittels der Hülsenlager 15 und der Schraubhülsen 22 höhenverstellbar lagerbar. Die Wandschlitzungen 44, dargestellt in den Figuren 10, 15 und 16 machen die Strebenhülsen 13, wie im Längsschnitt in der Figur 14 gezeigt ist, zu Spannhülsen 14, wobei die Zug-Spannschrauben 39 sowohl am Hülsenlager 15 als auch am Kugellager 16, in entsprechenden Querschnitten der Figuren 15 und 16 zur Darstellung gebracht, derartig klemmenden Kraftschluß aufbringen, daß ein verdrehfestes Haften der Kupplungsglieder 17 und der Schraubhülsen 22 in ihren jeweiligen Lagern erzeugt werden kann. Da die Schraubhülsen 22, wie die Figur 17 wiedergibt, geschlitzt ist, überträgt sich die Klemmkraft der Zug-Spannschrauben 39 auf die Streben-Spindel 12 in dem Maße, daß sogar eine durchgreifende Verdrehfestigkeit von der äußeren Spannhülse 14 bis zur inneren Streben-Spindel 12 eingestellt werden kann. So ist es dank der Schraubhülsen-Längsschlitze 23 möglich, auf gesonderte Kontermuttern zu verzichten, die vielfältige und erschwerende Montage-Hantierungen nach sich zögen.

Figur 18 stellt einen Längsschnitt einer alternativen Ausgestaltung der Streben-Spindeln 12 dar. Es kann dabei auf die Schraubhülse 22 verzichtet werden, da diese Spindel 12 - zur Gewichtseinsparung gehöhlt - mit einem zapfenförmig steckbaren Ende mit Ringnut 27 in die Rahmen-Querbalken 35 steckbar ist, und in dieser Form die Streben-Spindel 12 über eine Madenschraube 38 sowohl drehbar verriegelbar als auch drehfest arretierbar ist. Der Flansch 24 und die Stecklöcher 26 dienen hierbei wiederum der Übertragung der Schraubkräfte zur teleskopischen Längenverstellung der Streben 9.

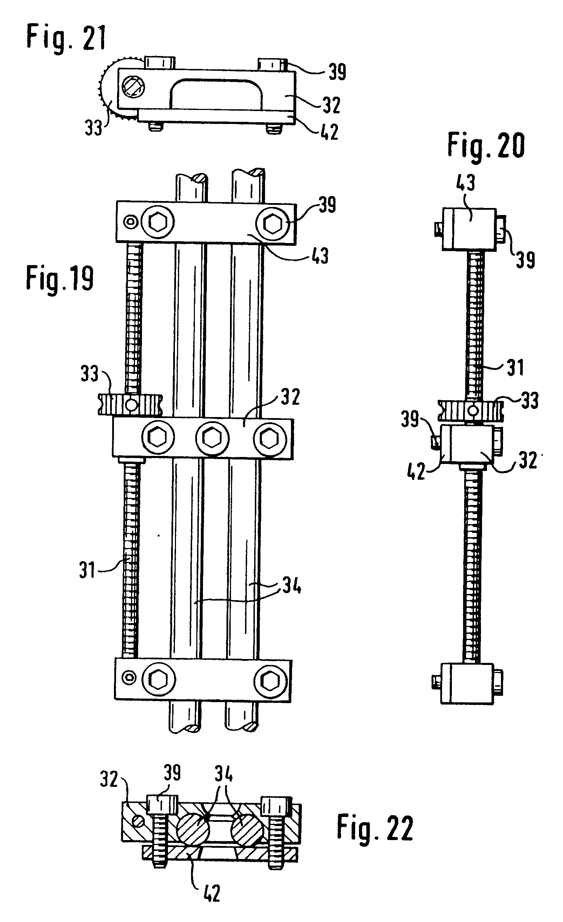

Die Figuren 19 - 22 geben verschiedene Ansichten bzw. Schnitte einer Transportiervorrichtung zur Höheneinstellung von an den Streben-Rahmenträgern 34 gelagerten Stangen-Drahtlagern 32 wieder. Querträger 43 haltern an den Rahmenträgern 34 die transportierende Spindel 31, auf welcher eine oder mehrere Stellmuttern 33 anbringbar sind. Diese unter Gewindebetätigung höhenverstellbaren Stellmuttern 33, an denen die Stangen-Drahtlager 32 gehalten sind, bewirken eine exakt einstellbare Verlagerung der Gewindedrähte 2. Aus solchen Abstandsänderungen der Drahtverankerungsorte von Drähten 2 unterschiedlicher Etagen des gleichen Knochenstückes werden Spannungsregulierungen der Drähte 2 erzielt, die ihrerseits eine relative Verschieblichkeit eines Knochenstückes gegenüber einem anderen benachbart liegenden Knochenstück bewirken können.

Auf den im Verlaufe der Strebenlängsachse 10 einen Längsspalt 11 bildenden rahmenförmigen Abschnitten der Streben 9 können auch ohne Einsatz einer Transportier-Vorrichtung Drahtlager 32 entweder verschieblich oder feststellbar gelagert werden. Bei über die teleskopischen Strebenlager 13 ausgeführten Änderungen der Strebenlängen mit verschieblich gehalterten Stangen-Drahtlagern 32 behalten mitwandernde Gewindedrähte 2 ihre Ausgangsspannung bei. Dagegen entstehen bei Feststllung der Stangen-Drahtlager 32 und gleichzeitiger Teleskop-Bedienung der Streben-Lager 13 je nach der Richtung der Teleskopbewegung bikonvexe oder bikonkave Seitausbiegungen der Gewindedrähte 2 zueinander, da die Längenänderung an den Streben 9 auf einem zwischen ihren Verankerungen gelegenen Höhenniveau vor sich geht. Dies führt ebenfalls zur relativen Veränderung der Verschieblichkeit eines Knochenstückes gegenüber dem benachbarten Knochenstück.

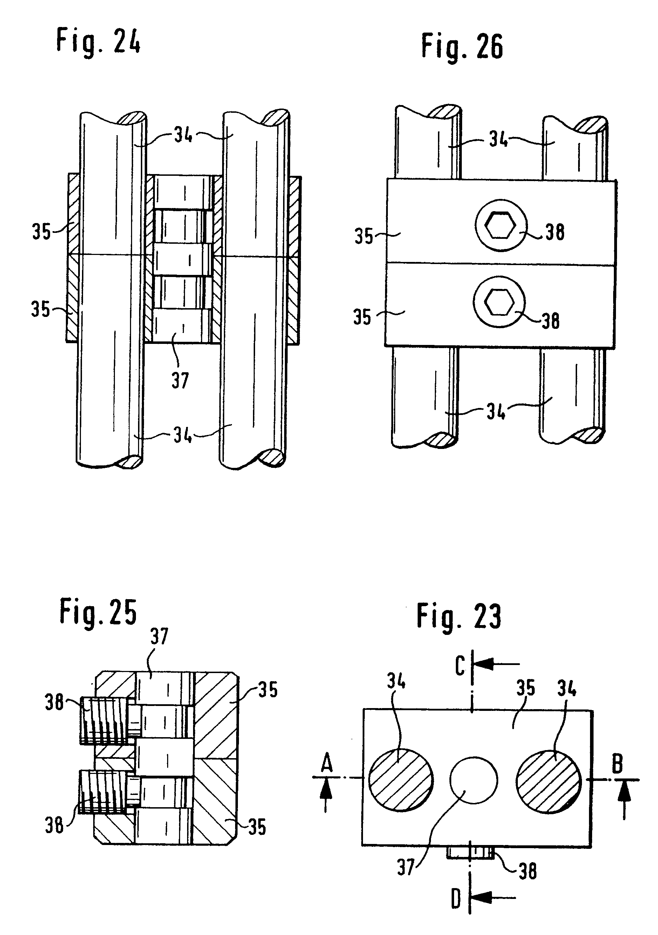

Die Figuren 23 - 26 geben verschiedene Ansichten und Schnitte der zusammensteck-, verriegel-, verdreh- und feststellbaren Verbindung von zwei rahmenförmigen Streben-Abschnitten miteinander wieder, wobei die Ausgestaltung und Funktionsweise der diese Strebenabschnitte verbindenden Kupplungsachse 37 gezeigt wird. Die relative Verdrehbarkeit zweier derart aneinander gekoppelter Strebenabschnitte führt zu der Möglichkeit, dieselben in von einander verschieden gedrehte Positionen zu bringen und so auf unterschiedlichem Höhenniveau horizontale Drähte in divergente Richtungen knochentransfixierend bohren zu können.

Die Figuren 27 und 28 zeigen, daß auch durch Abwinkelung 36 der Kupplungsglieder 17 die orthograd-zylindrischen Montageformen des Ringfixateurs abgewandelt und daß damit zusätzliche und vermehrt schwenkbare Kompositionen verwirklicht werden können.

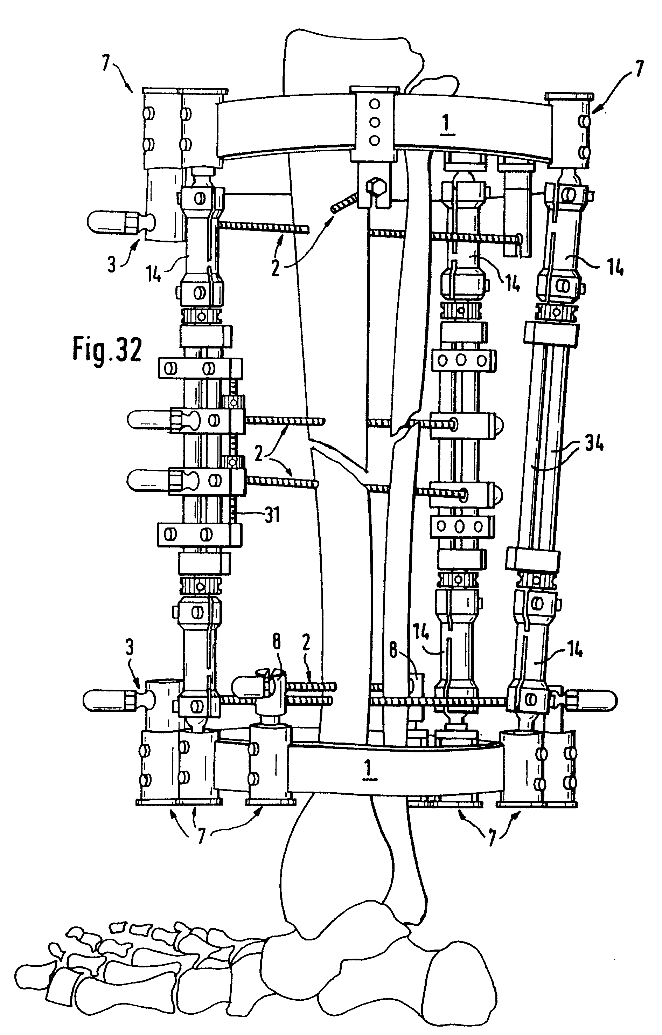

Die Figur 32 bildet in handgezeichneter Seitansicht unter Beibehaltung der bisher verwendeten Bezugszeichen einen am Unterschenkel montierten Ringfixateur aus zwei Ringen 1 und drei Streben 9 unter Verwendung von zwei Transportiervorrichtungen ab. Aus dieser Figur ist die räumliche Veränderbarkeit sämtlicher Teile zu einander ersichtlich. Ebenso ist aus dieser Figur offensichtlich, daß es sich beim erfindungsgemäßen Ringfixateur um einen Baukasten oder einen Bausatz handelt, der in weiten Bereichen veränderbar ist.

## Gewerbliche Anwendbarkeit

Der erfindungsgemäße Ringfixateur ist in der Technik der knochenchirurgischen Disziplinen für eine große Anzahl von Aufgabenstellungen anwendbar.
Er dient der Knochen-, Gelenk- und Weichteilbehandlung sowohl im stationären Klinikbereich, wie auch im operativen bzw. nachsorgenden Ambulanzbereich.
In der Unfallchirurgie, in der Orthopädie, in der Wiederherstellungschirurgie, aber auch in der Katastrophen-, Wehr- und Dritte-Welt-Medizin kann dieser Ringfixateur besonders zur Behandlung von Knochenbrüchen der Gliedmaßen, zur Gliedmaßenverlängerung, zur Ausheilung von Falschgelenken, zur KnochenNeuformung bei angeborenen oder erworbenen Knochendefekten, zur Korrektur von Achsenfehlstellungen der Gliedmaßen und der großen Körpergelenke, zur Ausheilung von Knochenmarkeiterungen, zur Remobilisation eingesteifter Gelenke, sowie zur Akutbehandlung bei Massenunfällen und in KatastrophenLagen zur Anwendung kommen, wobei in letzterem Fall die Spezifität in der Anpassungsfähigkeit an irreguläre Behelfssituationen mit Wegfall stationärer Patientenbehandlung und Einsparung von Vollnarkosen gelegen ist.
Eine besondere Einsatzmöglichkeit liegt in der gefahrlosen Hilfsfunktion dieses Ringfixateurs bei der Behandlung verstümmelnder Schwerstverletzungen, wie sie durch Explosions und Geschoßeinwirkungen hervorgerufen werden.

### Liste der Bezugszeichen

1 Ringe (Ringabschnitte)
2 Gewindedrähte
3 Draht-Spannmuttern
4 Draht-Positionsmuttern
5 Drahtgewinde
6 Draht-Lager
7 Ring-U-Halter
8 Loch-Lager
9 Streben
10 Streben-Achse
11 Streben-Spalt
12 Streben-Spindeln
13 Streben-Lager
14 Spannhülsen
15 Hülsenlager
16 Kugellager
17 Kupplungsglieder
18 Polyeder-Flächen
19 Kugelflächen der Draht-Spannmuttern
20 Gewinde-Enden der Draht-Spannmuttern
21 Schutzkappen
22 Schraubhülsen
23 Längsschlitze der Schraubhülsen
24 Flansche der Schraubhülsen und Spindeln
25 Gewinde-Abschnitte der Schraubhülsen und Spindeln
26 Lochkränze der Schraubhülsen und Spindeln
27 Ringnute an allen Teilen
28 Verriegelungsstecker am Ring
29 Tragplatte des Verriegelungssteckers
30 Zapfen des Verriegelungssteckers
31 Gewindespindeln der Streben
32 Drahtlager der Stangen
33 Stellmuttern der Transportiervorrichtung
34 Rahmenträger der Streben
35 Rahmen-Querbalken der Streben
37 Kupplungsachsen der rahmenförmigen Strebenabschnitte
36 Abwinkelung der Kupplungsglieder
38 alle Madenschrauben zum Verriegeln und Klemmen
39 alle Zug-Spannschrauben
40 alle sphärischen Lagerschalen
41 Einschlitzungen der Lochlager
42 Konterplatten der Streben-Drahtlager
43 Querträger der Transportierspindeln
44 Wandschlitzungen der Spannhülsen
45 Perforation der Tragplatte

### Patentansprüche

1. Ringfixateur zusammengesetzt aus Teilen (1 bis 17) eines Bausatzes zum Einrichten, Befestigen und Regulieren der Spannlage von Knochenabschnitten, bestehend aus durch diese Knochenabschnitte bohrbaren Drähten (2, 5), die zum Aufschrauben von Spann- (3) und Positionsmuttern (4) ausgebildet sind und aus einem mit den Drähten (2, 5) verbundenen zylindrischen Rahmengestell bestehend aus Ringen (1) und/oder Ringabschnitten in übereinanderliegenden Etagen, die durch in einstell- und veränderbaren Abständen und Neigungswinkeln angeordnete Streben (9) verbunden sind, die an den Enden drehbar gelagerte und verdrehfest klemmbare spindelförmige Abschnitte (12) aufweisen und aus die Verbindungen dieser Bauteile (1,2,5,7,9) herstellenden Befestigungsmitteln bestehend aus U-förmigen Reitern (7), die auf dem Umfang der Ringe (1) lösbar verschieblich und klemmbar gehaltert sind und mit auf den Reitern (7) in steckbarer Weise gehalterten Lochlagern (8), die auf den Reitern schwenkbar gehalten und um ihre Längsachse drehbar sind zum schwenkbaren Haltern und Spannen der in jeder Drehstellung befestigbaren Gewindedrähte (2,5), gekennzeichnet durch folgende Merkmale:

a) die Ringe (1) und die Ringteile sind im Wandquerschnitt hochformatig-rechteckig oder quadratisch,

b) die Drähte sind als Gewindedrähte (2,5) mit durchgehend sich erstreckenden Gewinden (5) ausgebildet,

c) die U-förmigen Reiter (7), die ebenso der Verbindung mit den Streben (9) dienen, sind radial von außen auf den Umfang der Ringe (1) in Ringradius-Richtung und/oder in einer hierzu um 90 Grad gedrehten Richtung steckbar und/oder auf den Streben (9) in klemmbarer Weise halterbar,

d) die Streben (9) zum Verbinden der Ringe (1) und/oder Ringteile sind zur durchsteckbaren Halterung und Spannung der Drähte (2, 5) abschnittweise mit in der Strebenachse (10) angeordneten spaltförmigen Durchlässen (11) ausgebildet, wobei die spindelförmigen Enden (12) der Streben (9) innerhalb des montierten Gesamtverbundes des Ringfixateurs austauschbar sind,

e) die Strebenlager (13) zum Verbinden der Strebenenden (12) mit den auf den Ringen (1)und-/oder Ringabschnitten befindlichen U-Reitern (7) weisen zwei beabstandete Lager (15;16) und Wandschlitzungen (44) auf zum jeweils separat verstell- und flächenschlüssig klemmbaren Befestigen von Kupplungsgliedern (17) der U-Reiter (7) einerseits und spindelförmigen Enden (12) der Streben (9) andererseits.

2. Ringfixateur nach Anspruch 1, dadurch gekennzeichnet,
daß Positionsmuttern (4) zum Aufschrauben auf die Gewindedrähte (2, 5) (nach Merkmal 1b) vorhanden sind, die polyederförmig-kantige Oberflächen (18) für auf den Drähten verdrehsichere gegenseitige Konterung und für linien- oder flächenschlüssiges Anlagern an Knochenwänden aufweisen.

3. Ringfixateur nach Anspruch 1, dadurch gekennzeichnet,
daß Draht-Spannmuttern (3) mit einer sphärischen Auflagefläche (19) und endständigem Gewinde (20) zum Anschrauben von Schutzkappen (21) ausgebildet sind.

4. Ringfixateur nach Anspruch 1, dadurch gekennzeichnet,
daß Schraubhülsen (22) zum Aufstecken und verdrehbaren Haltern auf den Spindelenden (12) der Streben (9) (nach Merkmal 1d) vorhanden sind, die mit einem Gewindeabschnitt (25) und mit wenigstens zwei Längsschlitzen (23) zur Überleitung von Zangenspannkräften der außenliegenden Spannhülsen (14) auf die innenliegenden Spindeln (12) versehen sind.

5. Ringfixateur nach Anspruch 1, dadurch gekennzeichnet,
daß die Spindeln (12) (nach Merkmal 1d) mit einem Flansch (24), mit einem Gewindeabschnitt (25), mit einer Schlüsselanflächung und/oder einem Schlüssel-Stecklochkranz (26) am Flanschumfang und mit einer Ringnut (27) für den Eingriff von die Verriegelung oder Verdrehsicherung aufbringenden Schrauben vorhanden sind.

6. Ringfixateur nach Anspruch 1, dadurch gekennzeichnet,
daß Verriegelungsstecker (28) als zwei auf einer Tragplatte (29) lotrecht befestigte, an ihren freien Enden geschlitzten Zapfen (30) vorhanden sind.

7. Ringfixateur nach Anspruch 1, dadurch gekennzeichnet,
daß in parallel axialer Anordnung an den Streben (9) befestigbare Gewindespindeln (31) und auf ihnen abschraubbare, die Höhenstellung von Drahtlagern (32) bestimmende Stellmuttern (33) angeordnet sind.

8. Ringfixateur nach Anspruch 1, dadurch gekennzeichnet,
daß die Streben (9) (nach Merkmal 1d) als Rahmen aus zwei längsachsenparallelen Trägern (34) und dazu rechtwinkelig gestellten Rahmen-Querbalken (35) mit austauschbaren, sowie verriegel- und klemmbaren Spindeln (12) verbindbar ausgebildet sind.

9. Ringfixateur nach Anspruch 1, dadurch gekennzeichnet,
daß die Kupplungsglieder (17) (nach Merkmal 1e) in ihrer Längsachse abgewinkelt (36) sind.

10. Ringfixateur nach Anspruch 1 und 8, dadurch gekennzeichnet,
daß Kupplungsachsen (37) zum Zusammenstecken und gegensinnigen Verdrehen von Strebenabschnitten (34,35) vorhanden sind, die steck- und drehbar und zum Eingriff von Schrauben (38) verriegelbar und formschlüssig klemmbar mit einer Ringnut (27) ausgebildet sind.

11. Ringfixateur nach Anspruch 1, dadurch gekennzeichnet,

daß die Ringe (1) und/oder die Ringteile aus in Umfangsrichtung endlos unidirektional gewickelten, in Matrix gebetteten Fasern mit einer Kunststoffummantelung ausgebildet sind.

## Claims

1. Ring splint consisting of parts (1 through 17) of a construction set to set, affix and regulate the tension position of bone segments, consisting of wires (2, 5) which can be drilled through these bone segments, which are designed to be screwed onto tension nuts (3) and position nuts (4) and, connected to these wires (2, 5), of a cylindrical frame consisting of rings (1) and/or ring sections in levels one above the other, which are connected by struts (9) arranged at adjustable and changeable intervals and angles and which, at their ends, have rotatably connected and non-twisting, clamping spindle-shaped sections (12), and consisting of the connections of the attachment mechanisms consisting of U-shaped riders (7) which form these components (1, 2, 5, 7, 9) and which are installed detachably along the circumference of the ring in such a way that it is possible to slide or clamp them, and with perforated bearings (8) that are attached on the riders (7) by insertion, in such a way that they can pivot and rotate around their longitudinal axis for purposes of pivoting attachment and tightening of the threaded wires (2, 5) in every rotational position, characterized by the following features:
    a) the wall cross section of the rings (1) and of the ring sections are upright-rectangular or square,
    b) the wires are designed as threaded wires (2, 5) with continuous threads (5),
    c) the U-shaped riders (7), which also serve to make the connection with the struts (9), can be placed radially from the outside onto the circumference of the rings (1) in the ring radius direction and/or in a direction rotated by 90 degrees to this and/or on the struts (9) so as to be clamped,
    d) for the penetrating attachment and tension of the wires (2, 5), sections of the struts (9) for connecting the rings (1) and/or the ring sections are designed with slit-like gaps (11) arranged in the strut axis (10), whereby the spindle-shaped ends (12) of the struts (9) are replaceable within the mounted overall assembly of the ring splint,
    e) the strut bearings (13) to connect the strut ends (12) with the U-riders (7) located on the rings (1) and/or ring sections have two bearings (15, 16) at a distance from each other as well as wall slits (44) for purposes of separate adjustable and form-fit clamping attachment of coupling elements (17) of the U-riders (7) on the one hand, and spindle-shaped ends (12) of the struts (9) on the other hand.

2. Ring splint according to Claim 1, characterized in that
there are position nuts (4) to be screwed onto the threaded wires (2, 5) (according to characteristic 1b), which have surfaces with polyhedron-shaped, angled surfaces (18) for countering each other on the wires so as to be non-twisting and for line and surface engaging positioning on bone walls.

3. Ring splint according to Claim 1, characterized in that
the wire tension nuts (3) are designed with a spherical bearing surface (19) and a threaded end section (20) so that protective caps (21) can be screwed on.

4. Ring splint according to Claim 1, characterized in that
there are screw sleeves (22) that can be inserted and rotating fasteners on the spindle ends (12) of the struts (9) (according to characteristic 1d), which are provided with a threaded section (25) and with at least two longitudinal slits (23) to transmit chuck-like clamping forces from the tension sleeves (14) located on the outside to the spindles (12) on the inside.

5. Ring splint according to Claim 1, characterized in that
the spindles (12) (according to characteristic 1d) have a flange (24), a threaded section (25), a surface to receive a key and/or a set of key-insertion holes (26) on the circumference of the flange and a ring groove (27) to engage with the screws that effectuate locking or prevent twisting.

6. Ring splint according to Claim 1, characterized in that
there are locking pins (28) in the form of two pivots (30) which are affixed vertically on a carrier plate (29) and which are slit at their free ends.

7. Ring splint according to Claim 1, characterized in that
there are threaded spindles (31) which can be attached to the struts (9) in a parallel axial arrangement, and there are adjustment nuts (33) which can be screwed onto these spindles and which can adjust the height of the wire bearings (32).

8. Ring splint according to Claim 1, characterized in that
the struts (9) (according to characteristic 1d) are designed as a frame consisting of two carriers (34) parallel to the longitudinal axis and, positioned at right angles to this, frame transverse bars (35) that can be connected to replaceable as well as locking and clamping spindles (12).

9. Ring splint according to Claim 1, characterized in that
the longitudinal axis of the coupling elements (17) (according to characteristic 1e) is at an angle.

10. Ring splint according to Claims 1 and 8, characterized in that
there are coupling axes (37) to put together and turn strut sections (34, 35) in the opposite direction, which are designed so that, for engaging with screws (38), it is possible to fasten and rotate them as well as to lock and clamp them in a form-fit manner with a ring groove (27).

11. Ring splint according to Claim 1, characterized in that
the rings (1) and/or the ring sections are made of fibers having a plastic sheathing which are continuously wound unidirectionally in the direction of the circumference and which are embedded in a matrix.

## Revendications

1. Attelle à anneaux composée d'éléments (1 à 17) d'un jeu de pièces destinées à redresser, fixer et régler la position de tension de segments d'os, comprenant des fils métalliques (2, 5) pouvant être transpercés à travers ces segments d'os et sur lesquels des écrous tendeurs (3) et de positionnement (4) peuvent être vissés, et d'un cadre cylindrique relié aux fils (2, 5) comprenant des anneaux (1) et/ou des sections d'anneaux disposés à des niveaux superposés et reliés entre eux par des barres (9) disposées à des distances et à des angles d'inclinaison variables et modifiables et dont les extrémités sont munies de sections (12) fusiformes logées de façon pivotante et serrables afin de résister à la torsion ainsi que de moyens de fixation réalisant l'assemblage de ces éléments (1,2,5,7,9) et consistant en des cavaliers en U (7) fixés sur le pourtour des anneaux (1) de manière coulissante et serrable, avec des paliers perforés (8) fixés sur les cavaliers (7) de façon embrochable et pouvant tourner autour de leur axe longitudinal pour fixer de façon pivotante et pour tendre les fils filetés (2,5) qui peuvent être arrêtés dans n'importe quelle position de rotation, caractérisée par les faits suivants:

a) les anneaux (1) et les parties d'anneaux présentent une section de paroi ayant la forme d'un rectangle en hauteur ou d'un carré,

b) les fils sont des fils filetés (2,5) avec des filets s'étendant en continu (5),

c) les cavaliers en U (7) servant également à réaliser l'assemblage avec les barres (9), peuvent être embrochés radialement de l'extérieur sur le pourtour des anneaux (1) en direction de leur rayon et/ou dans une direction orientée à 90° par rapport à celui-ci, et/ou peuvent être serrés sur les barres (9),

d) pour assurer le passage, la fixation et la tension des fils (2,5), les barres (9) servant à relier les anneaux (1) et/ou les parties d'anneaux présentent des passages (11) en forme de fente, les extrémités fusiformes (12) des barres (9) pouvant être échangées dans l'ensemble de l'attelle à anneaux monté,

e) les paliers des barres (13) servant à relier les extrémités des barres (12) avec les cavaliers en U (7) disposés sur les anneaux (1) et/ou sur les sections d'anneaux présentent deux paliers distants (15;16) et des fentes (44) dans la paroi servant à la fixation séparément réglable et serrable sur toute la surface d'organes d'accouplement (17) des cavaliers en U (7) d'une part et des extrémités fusiformes (12) des barres (9) d'autre part.

2. Attelle à anneaux suivant la revendication 1, caractérisée par le fait
qu'elle comporte des écrous de positionnement (4) à visser sur les fils filetés (2,5) (suivant la caractéristique 1b) et présentant des surfaces (18) polyédriques à arêtes assurant un blocage sûr à l'épreuve de la torsion sur les fils et un positionnement sur toute une ligne ou toute une surface aux parois de l'os.

3. Attelle à anneaux suivant la revendication 1, caractérisée par le fait
qu'elle comporte des écrous tendeurs des fils (3) avec une surface d'appui sphérique (19) et dont l'extrémité est pourvue d'un filet (20) permettant de visser des capots protecteurs (21) sur celle-ci.

4. Attelle à anneaux suivant la revendication 1, caractérisée par le fait
qu'elle comporte des douilles filetées (22) pouvant être mises et fixées par rotation sur les extrémités fusiformes (12) des barres (9) (selon la caractéristique 1d) et qui présentent une section filetée (25) et au moins deux fentes longitudinales (23) pour transmettre les forces de serrage des douilles de serrage extérieures aux broches (12) situées à l'intérieur.

5. Attelle à anneaux suivant la revendication 1, caractérisée par le fait
que les broches (12) (selon la caractéristique 1d) présentent une bride (24), une section filetée (25), une surface pouvant recevoir une clé et/ou une couronne à trous pour clé (26) sur le pourtour de la bride et une rainure annulaire (27) pour l'engrenage de vis assurant le verrouillage et la protection contre la torsion.

6. Attelle à anneaux suivant la revendication 1, caractérisée par le fait
qu'elle présente des fiches de verrouillage (28) en tant que deux pivots (30) fixés perpendiculairement sur une plaque de support (29) et dont les extrémités libres sont fendues.

7. Attelle à anneaux suivant la revendication 1, caractérisée par le fait
qu'elle comporte des broches filetées (31) pouvant être fixées sur les barres en disposition parallèle axiale et que sur lesdites broches sont disposés des écrous de réglage (33) dévissables déterminant la hauteur des paliers des fils.

8. Attelle à anneaux suivant la revendication 1, caractérisée par le fait
que les barres (9) (selon la caractéristiques 1d) se présentent sous forme d'un cadre comportant deux poutres (34) à axes longitudinaux parallèles et des traverses (35) disposées à angle droit par rapport à ceux-ci pouvant être reliés par des broches (12) échangeables, verrouillables et serrables.

9. Attelle à anneaux suivant la revendication 1, caractérisée par le fait

que les organes d'accouplement (17) (selon la caractéristique 1e) sont coudés dans leur axe longitudinal (36).

10. Attelle à anneaux suivant l'une quelconque des revendications 1 et 8, caractérisée par le fait

qu'elle présente des axes d'accouplement (37) pour assembler et tourner en sens inverse des sections de barres (34,35) et qui peuvent être embrochés et tournés ainsi que verrouillés pour l'engrenage de vis (38) et serrés à bloc avec une rainure annulaire (27).

11. Attelle à anneaux suivant la revendication 1, caractérisée par le fait

que les anneaux (1) et/ou les parties d'anneaux sont réalisés en fibres continues enroulées unidirectionnellement en sens circonférentiel et noyées dans une matrice, le tout étant doté d'une gaine en matière plastique.

Fig.1

Fig. 2

Fig.3

Fig.4

Fig.5

Fig. 6

Fig. 7

Fig. 8

Fig. 9

Fig. 10

Fig.11

Fig.12

Fig.13

**Fig. 17**

**Fig. 15**

**Fig. 18**

**Fig. 14**

**Fig. 16**

**Fig. 21**

39

33

32

42

**Fig. 20**

43

39

**Fig. 19**

39

43

33

32

31

34

31

33

39

42

32

**Fig. 22**

32   39   34

42

**Fig. 24**

**Fig. 26**

**Fig. 25**

**Fig. 23**

Fig. 27

17

27

7

Fig. 31

5    2    8

41

A          B

40

27

Fig. 29

Fig. 28

27

36

17

41

Fig. 30

40

27

Fig.32